(19) Europäisches Patentamt
European Patent Office
Office européen des brevets

(11) **EP 1 809 347 B1**

(12) **EUROPEAN PATENT SPECIFICATION**

(45) Date of publication and mention of the grant of the patent:
**17.02.2016 Bulletin 2016/07**

(21) Application number: **05817081.2**

(22) Date of filing: **25.10.2005**

(51) Int Cl.:
*A61L 29/14* *(2006.01)*   *A61L 29/16* *(2006.01)*
*A61L 31/14* *(2006.01)*   *A61L 31/16* *(2006.01)*
*A61L 27/50* *(2006.01)*

(86) International application number:
**PCT/US2005/038324**

(87) International publication number:
**WO 2006/049942 (11.05.2006 Gazette 2006/19)**

(54) **DRUG COATED MEDICAL DEVICE WITH NUCLEATING AGENTS**

ARZNEIMITTEL-BESCHICHTETES MEDIZINISCHES GERÄT MIT NUKLEIERUNGSMITTEL

DISPOSITIF MEDICAL ENROBE D'AGENTS DE NUCLEATION

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.10.2004 US 973305**

(43) Date of publication of application:
**25.07.2007 Bulletin 2007/30**

(73) Proprietor: **Boston Scientific Limited Hamilton HM11 (BM)**

(72) Inventors:
• **PARSONAGE, Edward**
  **St. Paul, MN 55116 (US)**
• **KANGAS, Steve**
  **Woodbury, MN 55125 (US)**
• **GIRTON, Timothy, S.**
  **Edina, MN 55436 (US)**

(74) Representative: **Peterreins Schley Patent- und Rechtsanwälte Soeltlstraße 2a 81545 München (DE)**

(56) References cited:
WO-A-01/13819        WO-A-2005/012413
WO-A-2005/079754     WO-A-2005/079884
WO-A2-98/43618       US-A1- 2002 133 183
US-A1- 2002 165 608

## Description

FIELD OF THE INVENTION

The present invention is directed to a method of controlling the release of drug particles from the surface of a coated medical device by adding nucleating agents to the coating of the medical device.

BACKGROUND OF THE INVENTION

Minimally invasive medical devices such as stents, grafts, and balloon catheters, are used for a number of medical purposes. It is often beneficial to add coatings containing drugs to such medical devices to provide desired therapeutic properties and effects. For example, it is useful to apply a coating containing drugs to medical devices to provide for the localized delivery of drugs to target locations within the body. Compared to systemic drug administration, such localized drug delivery minimizes unwanted effects on parts of the body that are not to be treated and allows for the delivery of higher amounts of drugs to the afflicted part of the body.

An important consideration in the manufacture of medical devices having a coating containing drugs is obtaining the desired release rate of the drugs from the coating. Current factors that affect drug release and that are therefore modulated during the medical device development process to affect drug release from a coating include polymer characteristics, drug loading, solvent selection, and variables in the coating process such as solution flow rate, nitrogen pressure, temperature, and humidity. For coatings applied by a spray process, varying any of the spray process factors within current manufacturing limits typically has a relatively small impact on the kinetic drug release of the drug. Currently, the primary way to substantially affect the kinetic drug release of drug particles from a coating is to modulate the amount of drug in the coating. However, simply adding more or less drug to the coating to affect the rate of drug release from the surface of the coating can create unwanted effects on the subsequent release of drug embedded in the polymer matrix of the coating, such as higher or lower drug release than desired. Furthermore, adding more drug to the coating may not be a cost-efficient mechanism to increase the drug release considering the high cost of many of the drugs that are incorporated into the coating.

International Patent Application Publication WO 01/13819 A2 purportedly describes a method of making a biodegradable polymeric implant. Crystal-nucleating agents may be introduced into the monomeric mixture prior to the formation of the polymer. Biologically active substance may be introduced in the mixing step of the process. U.S. Patent Application Nos. US 2002/0165608 A1 and US 2002/0133183 A1 purportedly describe medical devices coated with at least one agent in therapeutic dosage incorporated into a polymeric matrix. A lubricant or mold release agent may be utilized to prepare a lubricious coating onto or into the polymer matrix. Such lubricants and mold release agents include various polymeric and inorganic materials such as silicones and mica. International Patent Application Publication WO 2005/079754 A1 describes medical articles comprising a release region, which comprises (a) polymeric carrier comprising a polymer and (b) drug loaded nanoparticles. The drug loaded nanoparticles comprise a layered silicate material and a therapeutic agent. International Patent Application Publication WO 2005/079884 A1 describes medical articles comprising (a) a therapeutic agent and (b) a release region comprising (i) a polymer and (ii) a filler comprising inorganic platelet particles. International Patent Application Publication WO 2005/012413 A purportedly describes a crystal nucleating agent composition comprising a crystal nucleating agent, a lithium salt of an organic fatty acid, and a metal salt of an organic fatty acid. The crystal nucleating agent composition is used to form a crystalline polymer composition.

Accordingly, there is a need in the art for a more efficient and precise method of controlling the rate of drug release from the surface of a coated medical device.

SUMMARY OF THE INVENTION

In an embodiment, the present invention provides a medical device having a coating on at least a portion thereof. The coating comprises a polymer, a drug, and a nucleating agent having a particle radius greater than the critical radius for particle growth.

In another embodiment, the present invention provides a method of increasing the size of drug particles in a coating on a substrate comprising providing a substrate and preparing a mixture comprising a polymer, a solvent, drug particles, and nucleating agents that decrease the nucleation rate of the drug particles. The method further comprises applying the mixture to the substrate to form a coating on the substrate and allowing the drug particles to bind to the nucleating agents.

In another embodiment, the present invention provides a method of decreasing the size of drug particles in a coating on a substrate comprising providing a substrate and preparing a mixture comprising a polymer, a solvent, drug particles, and nucleating agents that increase the nucleation rate of the drug particles. The method further comprises applying the mixture to the substrate to form a coating on the substrate and allowing the drug particles to bind the nucleating agents.

DETAILED DESCRIPTION OF THE INVENTION

In an embodiment, the present invention provides a medical device having a coating that comprises a polymer, a drug, and a nucleating agent that increases or decreases the nucleation rate of the drug. As understood by one of skill in the art, the nucleation rate is the number of drug particles that form in the polymer per unit of time. Such effect on the nucleation rate of the drug can increase or decrease the size and number of the drug particles and therefore affect the release rate of the drug from the coating. In order for nucleation to occur, the nucleating agent, according to the present invention, has a particle radius of the following formula:

$$(R) > \frac{2(s)}{(G)}$$

wherein (R) is the particle radius of the nucleating agent, (s) is the drug/solution surface tension and (G) is the drug energy of formation. To increase the nucleation rate of the drug, a nucleating agent can be chosen that decreases the surface tension or increases the formation enthalpy of the drug, for example. To decrease the nucleation rate of the drug, a nucleating agent can be chosen that increases the surface tension or decreases the formation enthalpy of the drug, for example.

In another embodiment, the present invention provides a method of increasing or decreasing the size of drug particles in a coating on a substrate comprising preparing a mixture comprising a polymer, a solvent, drug particles, and nucleating agents. If it is desired to increase the size of the drug particles, then nucleating agents are used that decrease the nucleation rate of the drug particles. If it is desired to decrease the size of the drug particles, then nucleating agents are used that increase the nucleation rate of the drug particles. The mixture is then applied to the substrate to form a coating on the substrate. The drug particles are allowed to bind to the nucleating agents to increase or decrease size of the drug particles in the coating (depending on the nucleating agent).

The methods of this embodiment of the present invention can also affect the number of drug particles in the coating. Specifically, nucleating agents that increase the size of the drug particles also decrease the number of the drug particles whereas nucleating agents that decrease the size of the drug particles increase the number of the drug particles. The methods of this embodiment also provide a mechanism by which to control the release rate of the drug particles from the coating. Specifically, a method where the nucleating agents decrease the size of the drug particles results in a decrease in the release rate of the drug particles from the substrate. A method where the nucleating agents increase the size of the drug particles results in an increase in the release rate of the drug particles from the substrate. In a preferred embodiment, the substrate is a medical device.

As stated earlier, the nucleating agent according to the present invention can be any nucleating agent having a particle radius of the following formula:

$$(R) > \frac{2(s)}{(G)}$$

wherein (R) is the particle radius of the nucleating agent, (s) is the drug/solution surface tension and (G) is the drug energy of formation. Such nucleating agents include polymers and compounds. Non-limiting examples of nucleating agent are nanoparticles such as clays or micas; polyhedral oligomeric silsesquioxanes carbon or ceramic nano-tubes, nano-wires, or nano-fibers; nano-sized metal or metal oxide powders; nano-sized organic filler powders; and dendrimers. Non-limiting examples of clays or micas include montomorillonites, hectorites, hydrotalcites, vermiculites, and laponites. Non-limiting examples of polyhedral oligomeric silsesquioxanes include functionalized and/or polymerized polyhedral oligomeric silsequioxanes. Non-limiting examples of carbon or ceramic nano-tubes, nano-wires, or nano-fibers include single or multi-walled fullerene nano-tubes, silica nano-gels, and alumina nano-fibers. Non-limiting examples of nano-sized metal or metal oxide powders include aluminum oxide, titanium oxide, and magnetic nydmium iron boron. Non-limiting examples of nano-powdered organic fillers include polytetrafluoroethylene. Non-limiting examples of dendrimers include metal-dendrimer complexes.

The drug incorporated in the coating may be any pharmaceutically acceptable agent such as a non-genetic therapeutic agent, a biomolecule, a small molecule, or cells.

Exemplary non-genetic therapeutic agents include anti-thrombogenic agents such heparin, heparin derivatives, prostaglandin (including micellar prostaglandin E1), urokinase, and PPack (dextrophenylalanine proline arginine chloromethylketone); antiproliferative agents such as enoxaprin, angiopeptin, sirolimus (rapamycin), tacrolimus, everolimus, monoclonal antibodies capable of blocking smooth muscle cell proliferation, hirudin, and acetylsalicylic acid; anti-inflammatory agents such as dexamethasone, rosiglitazone, prednisolone, corticosterone, budesonide, estrogen, estrodiol, sulfasalazine, acetylsalicylic acid, mycophenolic acid, and mesalamine; anti-neoplastic/anti-proliferative/anti-mitotic agents such as paclitaxel, epothilone, cladribine, 5-fluorouracil, methotrexate, doxorubicin, daunorubicin, cyclosporine, cisplatin, vinblastine, vincristine, epothilones, endostatin, trapidil, halofuginone, and angiostatin; anti-cancer agents such as antisense inhibitors of c-myc oncogene; anti-microbial agents such as triclosan, cephalosporins, aminoglycosides, nitrofurantoin, silver ions, compounds, or salts; biofilm synthesis inhibitors such as non-steroidal anti-inflammatory agents and chelating agents such as ethylenediaminetetraacetic acid, O,O'-bis (2-aminoethyl)ethyleneglycol-N,N,N',N'-tetraacetic acid and mixtures thereof; antibiotics such as gentamycin, rifampin, minocyclin, and ciprofolxacin; antibodies including chimeric antibodies and antibody fragments; anesthetic agents such as lidocaine, bupivacaine, and ropivacaine; nitric oxide; nitric oxide (NO) donors such as lisidomine, molsidomine, L-arginine, NO-carbohydrate adducts, polymeric or oligomeric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, warfarin sodium, Dicumarol, aspirin, prostaglandin inhibitors, platelet aggregation inhibitors such as cilostazol and tick antiplatelet factors; vascular cell growth promotors such as growth factors, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogeneus vascoactive mechanisms; inhibitors of heat shock proteins such as geldanamycin; angiotensin converting enzyme (ACE) inhibitors; beta-blockers; bAR kinase (bARKct) inhibitors; phospholamban inhibitors; and any combinations and prodrugs of the above.

Exemplary biomolecules include peptides, polypeptides and proteins; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Nucleic acids may be incorporated into delivery systems such as, for example, vectors (including viral vectors), plasmids or liposomes.

Non-limiting examples of proteins include serca-2 protein, monocyte chemoattractant proteins ("MCP-1) and bone morphogenic proteins ("BMP's"), such as, for example, BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15. Preferred BMPS are any of BMP-2, BMP-3, BMP-4, BMP-5, BMP-6, and BMP-7. These BMPs can be provided as homdimers, heterodimers, or combinations thereof, alone or together with other molecules. Alternatively, or in addition, molecules capable of inducing an upstream or downstream effect of a BMP can be provided. Such molecules include any of the "hedghog" proteins, or the DNA's encoding them. Non-limiting examples of genes include survival genes that protect against cell death, such as anti-apoptotic Bcl-2 family factors and Akt kinase; serca 2 gene; and combinations thereof. Non-limiting examples of angiogenic factors include acidic and basic fibroblast growth factors, vascular endothelial growth factor, epidermal growth factor, transforming growth factor $\alpha$ and $\beta$, platelet-derived endothelial growth factor, platelet-derived growth factor, tumor necrosis factor $\alpha$, hepatocyte growth factor, and insulin like growth factor. A non-limiting example of a cell cycle inhibitor is a cathespin D (CD) inhibitor. Non-limiting examples of anti-restenosis agents include p15, p16, p18, p19, p21, p27, p53, p57, Rb, nFkB and E2F decoys, thymidine kinase ("TK") and combinations thereof and other agents useful for interfering with cell proliferation.

Exemplary small molecules include hormones, nucleotides, amino acids, sugars, and lipids and compounds have a molecular weight of less than 100kD.

Exemplary cells include stem cells, progenitor cells, endothelial cells, adult cardiomyocytes, and smooth muscle cells. Cells can be of human origin (autologous or allogenic) or from an animal source (xenogenic), or genetically engineered. Non-limiting examples of cells include side population (SP) cells, lineage negative (Lin$^-$) cells including Lin$^-$CD34$^-$, Lin$^-$CD34$^+$, Lin$^-$cKit$^+$, mesenchymal stem cells including mesenchymal stem cells with 5-aza, cord blood cells, cardiac or other tissue derived stem cells, whole bone marrow, bone marrow mononuclear cells, endothelial progenitor cells, skeletal myoblasts or satellite cells, muscle derived cells, go cells, endothelial cells, adult cardiomyocytes, fibroblasts, smooth muscle cells, adult cardiac fibroblasts + 5-aza, genetically modified cells, tissue engineered grafts, MyoD scar fibroblasts, pacing cells, embryonic stem cell clones, embryonic stem cells, fetal or neonatal cells, immunologically masked cells, and teratoma derived cells.

Any of the therapeutic agents may be combined to the extent such combination is biologically compatible.

Any of the above mentioned therapeutic agents may be incorporated into the polymeric coating on the substrate or medical device or applied onto a polymeric coating on the substrate or medical device. The polymers of the polymeric coatings may be biodegradable or non-biodegradable. Non-limiting examples of suitable non-biodegradable polymers include polystrene; polyisobutylene copolymers and styrene-isobutylene-styrene block copolymers such as styrene-isobutylene-styrene tert-block copolymers (SIBS); polyvinylpyrrolidone including cross-linked polyvinylpyrrolidone; polyvinyl alcohols, copolymers of vinyl monomers such as EVA; polyvinyl ethers; polyvinyl aromatics; polyethylene oxides; polyesters including polyethylene terephthalate; polyamides; polyacrylamides; polyethers including polyether sulfone; polyalkylenes including polypropylene, polyethylene and high molecular weight polyethylene; polyurethanes; polycarbonates, silicones; siloxane polymers; cellulosic polymers such as cellulose acetate; polymer dispersions such as polyurethane dispersions (BAYHDROL®); squalene emulsions; and mixtures and copolymers of any of the foregoing.

Non-limiting examples of suitable biodegradable polymers include polycarboxylic acid, polyanhydrides including maleic anhydride polymers; polyorthoesters; poly-amino acids; polyethylene oxide; polyphosphazenes; polylactic acid, polyglycolic acid and copolymers and mixtures thereof such as poly(L-lactic acid) (PLLA), poly(D,L,-lactide), poly(lactic acid-co-glycolic acid), 50/50 (DL-lactide-co-glycolide); polydioxanone; polypropylene fumarate; polydepsipeptides; polycaprolactone and co-polymers and mixtures thereof such as poly(D,L-lactide-co-caprolactone) and polycaprolactone co-butylacrylate; polyhydroxybutyrate valerate and blends; polycarbonates such as tyrosine-derived polycarbonates and arylates, polyiminocarbonates, and polydimethyltrimethylcarbonates; cyanoacrylate; calcium phosphates; polyglycosaminoglycans; macromolecules such as polysaccharides (including hyaluronic acid; cellulose, and hydroxypropylmethyl cellulose; gelatin; starches; dextrans; alginates and derivatives thereof), proteins and polypeptides; and mixtures and copolymers of any of the foregoing. The biodegradable polymer may also be a surface erodable polymer such as polyhydroxybutyrate and its copolymers, polycaprolactone, polyanhydrides (both crystalline and amorphous), maleic anhydride copolymers, and zinc-calcium phosphate.

Such coatings used with the present invention may be formed by any method known to one in the art. The nucleating agents and drug which are added to the polymer may be added in any particular order. For example, the drug may be initially added to the polymer, the polymer matrix then applied to the medical device and then the nucleating agents added to the polymer matrix. Alternatively, the drug and the nucleating agents are simultaneously or sequentially added to the polymer and the resulting suspension is applied to the medical device. Solvents may also be utilized in any order. For example, an initial polymer/solvent mixture can be formed and then the drug added to the polymer/solvent mixture. Alternatively, the polymer, solvent, and drug can be added simultaneously to form a mixture. The polymer/solvent/drug mixture may be a dispersion, suspension or a solution. The drug may also be mixed with the polymer in the absence of a solvent. The drug may be dissolved in the polymer/solvent mixture or in the polymer to be in a true solution with the mixture or polymer, dispersed into fine or micronized particles in the mixture or polymer, suspended in the mixture or polymer based on its solubility profile, or combined with micelle-forming compounds such as surfactants or adsorbed onto small carrier particles to create a suspension in the mixture or polymer. The nucleating agents can be added at any point to the mixture. Furthermore, multiple types of drug, nucleating agents, polymers, and/or solvents may be utilized.

The coating can be applied to the medical device or substrate by any known method in the art including dipping, spraying, rolling, brushing, electrostatic plating or spinning, vapor deposition, air spraying including atomized spray coating, and spray coating using an ultrasonic nozzle.

The medical device may also contain a radio-opacifying agent within its structure to facilitate viewing the medical device during insertion and at any point while the device is implanted. Non-limiting examples of radio-opacifying agents are bismuth subcarbonate, bismuth oxychloride, bismuth trioxide, barium sulfate, tungsten, and mixtures thereof.

Non-limiting examples of substrates or medical devices according to the present invention include polymeric films, catheters, guide wires, balloons, filters (*e.g.,* vena cava filters), stents, stent grafts, vascular grafts, intraluminal paving systems, implants and other devices used in connection with drug-loaded polymer coatings. Such medical devices may be implanted or otherwise utilized in body lumina and organs such as the coronary vasculature, esophagus, trachea, colon, biliary tract, urinary tract, prostate, brain, lung, liver, heart, skeletal muscle, kidney, bladder, intestines, stomach, pancreas, ovary, cartilage, eye, bone, and the like.

The foregoing description and examples have been set forth merely to illustrate the invention and are not intended as being limiting. Each of the disclosed aspects and embodiments of the present invention may be considered individually or in combination with other aspects, embodiments, and variations of the invention. In addition, unless otherwise specified, none of the steps of the methods of the present invention are confined to any particular order of performance. Modifications of the disclosed embodiments incorporating the spirit and substance of the invention may occur to persons skilled in the art and such modifications are within the scope of the present invention. Furthermore, all references cited herein are incorporated by reference in their entirety.

## Claims

1.    A medical device having a coating on at least a portion thereof, the coating comprising polymer, a drug, and a nucleating agent having a size such that:

$$(R) > 2(s)/(G)$$

wherein (R) is the particle radius of the nucleating agent, (s) is the surface tension of the drug, and (G) is the drug energy of formation, and wherein the drug is bound to the nucleating agent through nucleation.

2. The medical device of claim 1, wherein the nucleating agent is a polymer.

3. The medical device of claim 1, wherein the nucleating agent is a clay or a mica.

4. The medical device of claim 3, wherein the clay or mica is intercalated or exfoliated.

5. The medical device of claim 3, wherein the clay or mica is a montmorillonite, a hectorite, a hydrotalcite, a vermiculite, a laponite, or any combination thereof.

6. The medical device of claim 1, wherein the nucleating agent is a polyhedral oligomeric silsesquioxane.

7. The medical device of claim 6, wherein the polyhedral oligomeric silsesquioxane is functionalized or polymerized.

8. The medical device of claim 1, wherein the nucleating agent is a carbon or ceramic nano-tube, nano-wire, or nano-fiber.

9. The medical device of claim 8, wherein the carbon or ceramic nano-tube, nano-wire, or nano-fiber is a single wall fullerene nano-tube, a mult-walled fullerene nano-tube, a silica nano-gel, or an alumina nano-fiber.

10. The medical device of claim 1, wherein the nucleating agent is a nano-sized metal or metal oxide powder.

11. The medical device of claim 10, wherein the nano-sized metal or metal oxide powder is aluminum oxide, titanium oxide, gold, or magnetic neodymium iron boron.

12. The medical device of claim 1, wherein the nucleating agent is a nano-powdered organic filler.

13. The medical device of claim 12, wherein the nano-powdered organic filler is polytetrafluoroethylene.

14. The medical device of claim 1, wherein the nucleating agent is a dendrimer.

15. The medical device of claim 14, wherein the dendrimer is a metal dendrimer complex.

16. A method of increasing the size of drug particles in a coating on a substrate comprising:

providing a substrate;
preparing a mixture comprising a polymer, a solvent, drug particles, and nucleating agents that decrease the nucleation rate of the drug particles;
applying the mixture to the substrate to form a coating on the substrate; and
allowing the drug particles to bind to the nucleating agents to increase the size of the drug particles in the coating.

17. A method of increasing the release rate of drug particles from a coating on a substrate comprising the method of claim 16, wherein the increase in the size of the drug particles increases the release rate of the drug particles from the coating.

18. The method of claim 16, wherein the nucleating agents increase the surface tension or decrease the formation enthalpy of the drug particles.

19. A method of decreasing the size of drug particles in a coating on a substrate comprising:

providing a substrate;
preparing a mixture comprising a polymer, a solvent, drug particles, and nucleating agents that increase nucleation rate of the drug particles;
applying the mixture to the substrate to form a coating on the medical device; and
allowing the drug particles to bind to the nucleating agents to decrease the size of the drug particles in the coating.

20. A method of decreasing the release rate of drug particles from a coating on a substrate comprising the method of claim 19, wherein the decrease in the size of the drug particles decreases the release rate of the drug particles from the coating.

21. The method of claim 19, wherein the nucleating agents decrease the surface tension or increase the formation enthalpy of the drug particles

**Patentansprüche**

1. Eine mindestens in einem Teilbereich eine Beschichtung aufweisende Medizinvorrichtung, wobei die Beschichtung ein Polymer, einen Arzneistoff und ein kernbildendes Mittel umfasst und das kernbildendes Mittel eine Größe aufweist, so dass:

$$(R) > 2(s)/(G)$$

wobei (R) der Teilchenradius des kernbildenden Mittels ist, (s) die Oberflächenspannung des Arzneistoffs ist und (G) die Bildungsenergie des Arzneistoffs ist, und wobei der Arzneistoff an das kernbildende Mittel durch einen Kernbildungsvorgang gebunden ist.

2. Die Medizinvorrichtung nach Anspruch 1, wobei das kernbildende Mittel ein Polymer ist.

3. Die Medizinvorrichtung nach Anspruch 1, wobei das kernbildende Mittel ein Ton oder ein Glimmer ist.

4. Die Medizinvorrichtung nach Anspruch 3, wobei der Ton oder der Glimmer interkaliert oder abgeschält ist.

5. Die Medizinvorrichtung nach Anspruch 3, wobei der Ton oder der Glimmer ein Montmorillonit, ein Hectorit, Hydrotalcit, ein Vermiculit, ein Laponit, oder irgendeine Kombination davon ist.

6. Die Medizinvorrichtung nach Anspruch 1, wobei das kernbildende Mittel ein polyedrisches oligomeres Silsesquioxan ist.

7. Die Medizinvorrichtung nach Anspruch 6, wobei das polyedrische oligomere Silsesquioxan funktionalisiert oder polymersiert ist.

8. Die Medizinvorrichtung nach Anspruch 1, wobei das kernbildende Mittel ein Kohlenstoff- oder keramische Nanoröhre, ein Nanodraht, oder eine Nanofaser ist.

9. Die Medizinvorrichtung nach Anspruch 8, wobei die Kohlenstoff- oder keramische Nanoröhre, der Nanodraht, oder die Nanofaser eine einwandige Fullerennanoröhre, eine mehrwandige Fullerennanoröhre, ein Silicananogel oder eine Aluminiumoxidnanofaser ist.

10. Die Medizinvorrichtung nach Anspruch 1, wobei das kernbildende Mittel ein Metall in Nanogröße oder ein Metalloxidpulver in Nanogröße ist.

11. Die Medizinvorrichtung nach Anspruch 10, wobei das Metall in Nanogröße oder ein Metalloxidpulver in Nanogröße Aluminiumoxid, Titanoxid, Gold, oder magnetisches Neodymeisenbor ist.

12. Die Medizinvorrichtung nach Anspruch 1, wobei das kernbildende Mittel ein nanogepulverter organischer Füllstoff ist.

13. Die Medizinvorrichtung nach Anspruch 12, wobei der nanogepulverte organische Füllstoff ein Polytetrafluorethylen ist.

**14.** Die Medizinvorrichtung nach Anspruch 1, wobei das kernbildende Mittel ein Dendrimer ist.

**15.** Die Medizinvorrichtung nach Anspruch 14, wobei das Dendrimer ein Metalldendrimerkomplex ist.

**16.** Ein Verfahren zur Steigerung der Größe von Arzneistoffteilchen in einer Beschichtung auf einem Substrat, umfassend:

Bereitstellen eines Substrats;
Herstellen einer Mischung umfassend ein Polymer, ein Lösungsmittel, Arzneistoffteilchen, und kernbildende Mittel, welche die Kernbildungsgeschwindigkeit der Arzneistoffteilchen verringern;
wobei die kernbildenden Mittel eine Größe aufweisen, so dass:

$$(R) > 2(s)/(G)$$

wobei (R) der Teilchenradius des kernbildenden Mittels ist, (s) die Oberflächenspannung des Arzneistoffs ist und (G) die Bildungsenergie des Arzneistoffs ist;
wobei die Arzneistoffteilchen durch Bindung durch Kernbildung an die kernbildenden Teilchen gebildet werden; und
Aufbringen der Mischung auf das Substrat zur Bildung einer Beschichtung auf dem Substrat.

**17.** Ein Verfahren zur Steigerung der Freisetzungsgeschwindigkeit von Arzneistoffteilchen aus einer Beschichtung auf einem Substrat, umfassend das Verfahren nach Anspruch 16, wobei die Steigerung der Größe der Arzneistoffteilchen die Freisetzungsgeschwindigkeit der Arzneistoffteilchen aus der Beschichtung steigert.

**18.** Das Verfahren nach Anspruch 16, wobei die kernbildenden Mittel die Oberflächenspannung erhöhen oder die Bildungsenthalpie der Arzneistoffteilchen verringern.

**19.** Ein Verfahren zur Verringerung der Größe von Arzneistoffteilchen in einer Beschichtung auf einem Substrat, umfassend:

Bereitstellen eines Substrats;
Herstellen einer Mischung umfassend ein Polymer, ein Lösungsmittel, Arzneistoffteilchen, und kernbildende Mittel, welche die Kernbildungsgeschwindigkeit der Arzneistoffteilchen erhöhen;
wobei die kernbildenden Mittel eine Größe aufweisen, so dass:

$$(R) > 2(s)/(G)$$

wobei (R) der Teilchenradius des kernbildenden Mittels ist, (s) die Oberflächenspannung des Arzneistoffs ist und (G) die Bildungsenergie des Arzneistoffs ist;
wobei die Arzneistoffteilchen durch Bindung durch Kernbildung an die kernbildenden Teilchen gebildet werden; und
Aufbringen der Mischung auf das Substrat zur Bildung einer Beschichtung auf dem Substrat.

**20.** Ein Verfahren zur Veringerung der Freisetzungsgeschwindigkeit von Arzneistoffteilchen aus einer Beschichtung auf einem Substrat, umfassend das Verfahren nach Anspruch 19, wobei die Verringerung der Größe der Arzneistoffteilchen die Freisetzungsgeschwindigkeit der Arzneistoffteilchen aus der Beschichtung verringert.

**21.** Das Verfahren nach Anspruch 19, wobei die kernbildenden Mittel die Oberflächenspannung verringern oder die Bildungsenthalpie der Arzneistoffteilchen erhöhen.

**Revendications**

1. Dispositif médical ayant un revêtement sur au moins une partie de celui-ci, le revêtement comprenant un polymère, un médicament, et un agent de nucléation ayant une taille telle que :

$$(R) > 2(s)/(G)$$

dans lequel (R) est le rayon particulaire de l'agent de nucléation, (s) est la tension superficielle du médicament, et (G) est l'énergie de formation du médicament, et dans lequel le médicament est lié à l'agent de nucléation par nucléation.

2. Dispositif médical de la revendication 1, dans lequel l'agent de nucléation est un polymère.

3. Dispositif médical de la revendication 1, dans lequel l'agent de nucléation est une argile ou un mica.

4. Dispositif médical de la revendication 3, dans lequel l'argile ou le mica est intercalé ou exfolié.

5. Dispositif médical de la revendication 3, dans lequel l'argile ou le mica est une montmorillonite, une hectorite, une hydrotalcite, une vermiculite, une laponite, ou toute combinaison de celles-ci.

6. Dispositif médical de la revendication 1, dans lequel l'agent de nucléation est un silsesquioxane oligomère polyédrique.

7. Dispositif médical de la revendication 6, dans lequel le silsesquioxane oligomère polyédrique est fonctionnalisé ou polymérisé.

8. Dispositif médical de la revendication 1, dans lequel l'agent de nucléation est un nanotube, un nanofil ou une nanofibre de carbone ou de céramique.

9. Dispositif médical de la revendication 8, dans lequel le nanotube, le nanofil ou la nanofibre de carbone ou de céramique est un nanotube de fullerène monoparoi, un nanotube de fullerène multiparoi, un nanogel de silice, ou une nanofibre d'alumine.

10. Dispositif médical de la revendication 1, dans lequel l'agent de nucléation est une poudre de métal ou d'oxyde métallique de taille nanométrique.

11. Dispositif médical de la revendication 10, dans lequel la poudre de métal ou d'oxyde métallique de taille nanométrique est de l'oxyde d'aluminium, de l'oxyde de titane, de l'or, ou du néodyme-fer-bore magnétique.

12. Dispositif médical de la revendication 1, dans lequel l'agent de nucléation est une charge organique en nanopoudre.

13. Dispositif médical de la revendication 12, dans lequel la charge organique en nanopoudre est du polytétrafluoroéthylène.

14. Dispositif médical de la revendication 1, dans lequel l'agent de nucléation est un dendrimère.

15. Dispositif médical de la revendication 14, dans lequel le dendrimère est un complexe métal-dendrimère.

16. Procédé d'augmentation de la taille de particules de médicament dans un revêtement sur un substrat comprenant :

l'obtention d'un substrat ;
la préparation d'un mélange comprenant un polymère, un solvant, des particules de médicament, et des agents de nucléation qui diminuent la vitesse de nucléation des particules de médicament,
les agents de nucléation ayant une taille telle que :

$$(R) > 2(s)/(G)$$

où (R) est le rayon particulaire de l'agent de nucléation, (s) est la tension superficielle du médicament, et (G) est l'énergie de formation du médicament ;
les particules de médicament étant formées par liaison aux agents de nucléation par nucléation ; et
l'application du mélange au substrat pour former un revêtement sur le substrat.

**17.** Procédé d'augmentation de la vitesse de libération de particules de médicament d'un revêtement sur un substrat comprenant le procédé de la revendication 16, l'augmentation de la taille des particules de médicament augmentant la vitesse de libération des particules de médicament du revêtement.

**18.** Procédé de la revendication 16, dans lequel les agents de nucléation augmentent la tension superficielle ou diminuent l'enthalpie de formation des particules de médicament.

**19.** Procédé de diminution de la taille de particules de médicament dans un revêtement sur un substrat comprenant :

l'obtention d'un substrat ;
la préparation d'un mélange comprenant un polymère, un solvant, des particules de médicament, et des agents de nucléation qui augmentent la vitesse de nucléation des particules de médicament,
les agents de nucléation ayant une taille telle que :

$$(R) > 2(s)/(G)$$

où (R) est le rayon particulaire de l'agent de nucléation, (s) est la tension superficielle du médicament, et (G) est l'énergie de formation du médicament ;
les particules de médicament étant formées par liaison aux agents de nucléation par nucléation ; et
l'application du mélange au substrat pour former un revêtement sur le substrat.

**20.** Procédé de diminution de la vitesse de libération de particules de médicament d'un revêtement sur un substrat comprenant le procédé de la revendication 19, la diminution de la taille des particules de médicament diminuant la vitesse de libération des particules de médicament du revêtement.

**21.** Procédé de la revendication 19, dans lequel les agents de nucléation diminuent la tension superficielle ou augmentent l'enthalpie de formation des particules de médicament.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0113819 A2
- US 20020165608 A1
- US 20020133183 A1
- WO 2005079754 A1
- WO 2005079884 A1
- WO 2005012413 A